Europäisches Patentamt

(19) European Patent Office

Office européen des brevets

(11) Publication number: **0 432 965 A1**

# (12) EUROPEAN PATENT APPLICATION

(21) Application number: 90313257.9

(22) Date of filing: 06.12.90

(51) Int. Cl.⁵: **C07K 7/00,** C12N 15/63,
A61K 37/02

(30) Priority: 08.12.89 US 447746

(43) Date of publication of application:
19.06.91 Bulletin 91/25

(84) Designated Contracting States:
AT BE CH DE DK ES FR GB GR IT LI LU NL SE

(71) Applicant: SMITHKLINE BEECHAM
CORPORATION
One Franklin Plaza
Philadelphia Pennsylvania 19103 (US)
Applicant: The United States of America as
respresented by the Secretary of the Army
One Franklin Plaza
Philadelphia, Pennsylvania 19103 (US)
Applicant: BIOMEDICAL RESEARCH
INSTITUTE
12111 Parklawn Drive
Rockville, MD 20852 (US)

(72) Inventor: Gross, Mitchell Stuart
667 Pugh Road
Wayne, Pennsylvania 19087 (US)
Inventor: Gordon, Daniel Matthew
14100 Gaines Avenue
Rockville, Maryland 20853 (US)
Inventor: Hollingdale, Michael Richard
1615 15th Street
N.W. Washington, D.C. 20009 (US)

(74) Representative: Dalton, Marcus Jonathan
William et al
SmithKline Beecham, Corporate Patents,
Great Burgh, Yew Tree Bottom Road
Epsom, Surrey KT18 5XQ (GB)

(54) **Malaria vaccine.**

(57) Vaccines against infection by parasites of the genus Plasmodium, polypeptides useful as therapeutic agents to inhibit infection by malaria parasites, which polypeptides comprise immunogenic determinants from regions of a Plasmodium surface protein flanking a central repeat domain thereof and fewer than all repeating immunogenic determinants from the repeat domain; and methods for treating humans against malaria infection.

EP 0 432 965 A1

## MALARIA VACCINE

BACKGROUND OF THE INVENTION

This invention relates to vaccines against infection by parasites of the genus Plasmodium and, more particularly, to polypeptides useful as therapeutic agents to inhibit infection by malaria parasites, which polypeptides comprise immunogenic determinants from regions of a Plasmodium surface protein flanking a central repeat domain thereof and fewer than all repeating immunogenic determinants from the repeat domain ; and to methods for treating humans against malaria infection.

SUMMARY OF THE INVENTION

This invention relates generally to a polypeptide comprising one or more immunogenic determinants from a first region flanking a central repeat domain of a Plasmodium surface protein, one or more immunogenic determinants from a second region flanking the repeat domain and fewer than all or none of the repeating immunogenic determinants from the central repeat domain

In one embodiment, the invention relates to a polypeptide comprising at least one but fewer than all repeating immunogenic determinants of a Plasmodium surface protein repeat domain and one or more immunogenic determinants from regions of a Plasmodium surface protein flanking the repeat domain.

In another embodiment of the invention, the polypeptide comprises substantially all of the immunogenic determinants from the regions flanking the central repeat domain, and is devoid of immunogenic determinants from the central repeat domain. Alternatively, the polypeptide comprising substantially all immunogenic determinants from the flanking regions further comprises at least one but fewer than all immunogenic determinants from the central repeat domain.

Immunogenic determinants useful in the polypeptides of the present invention preferably include those present in the surface proteins of Plasmodium falciparum, P. vivax, P. malariae, and P. ovale.

In yet another embodiment of the invention, the polypeptide is genetically fused to a carrier protein, preferably a carrier protein which either enhances expression of the polypeptide or enhances the immunogenicity of the polypeptide, or both.

In a preferred embodiment, the polypeptides of the present invention comprise an immunogenic carrier protein, for example, 81 N-terminal amino acids of influenza virus nonstructural protein 1 ($NSI_{81}$), fused, via a synthetic linker, to a first flanking region of a Plasmodium circumsporozoite (CS) protein, which is itself fused to a second flanking region of the CS protein.

Such polypeptides may further comprise more than one but fewer than all immunogenic determinants from the CS protein central repeat domain, for example, the immunogenic determinant from the repeat domain comprising a tetrapeptide having the amino acid sequence (Asn-X-Y-Pro), wherein X is Ala or Val and Y is Asn or Asp. The immunogenic determinants from the central repeat domain may be positioned, for example, between the carrier protein and the first flanking region or between the first flanking region and the second flanking region.

Another aspect the present invention includes expression vectors encoding the polypeptides, vaccines comprised of the polypeptides ; methods for purifying the polypeptides ; and methods for treating humans against infection by malaria using the peptides.

Other aspects and advantages of the present invention are disclosed in the detailed description which follows.

DESCRIPTION OF THE PREFERRED EMBODIMENTS

The following amino acid abbreviations are used in this patent application :
Ala Alanine
Arg Arginine
Asn Asparagine
Asp Aspartic Acid
Cys Cysteine
Glu Glutamic Acid
Gln Glutamine
Gly Glycine
His Histidine

Ile Isoleucine
Leu Leucine
Lys Lysine
Met Methionine
Phe Phenylalanine
Pro Proline
Ser Serine
Thr Threonine
Trp Tryptophan
Tyr Tyrosine
Val Valine

The protozoan malaria parasite, Plasmodium, has a number of stage-specific proteins present on its outer cell surface. These surface proteins have been found to have three regions in common, namely, a central repeated epitope region or domain and paired flanking regions. The first flanking region of the pair is fused to the carboxy-terminus of the central repeat domain and the second flanking region of the pair is fused to the amino terminus of the central repeat domain.

The polypeptides of the present invention are derived from a portion of a Plasmodium surface protein which contains fewer than all or none of the immunogenic determinants of the central repeat domain between the first and second flanking regions and are expressed in quantities sufficient for use as therapeutic agents to inhibit infection by malaria parasites.

In other words, the polypeptides of the invention have fewer tandem repeats between the first and second flanking regions than are present in the wild-type repeat regions. Thus, in the case of a polypeptide for protecting a human against infection by P. falciparum, the polypeptide may comprise the entire first flanking region, that is, the N-terminal flanking region of the P. falciparum circumsporozoite protein (PfCSP), the entire second flanking region, that is, the C-terminal flanking region from the PfCSP and, between the first and second flanking regions, less than 41 tandem repeats from the PfCSP, that is, less than 41 tetrapeptides of the formula Asn-X-Y-Pro, wherein X is Ala or Val and Y is Asp or Asn.

In a wild-type Plasmodium surface protein, the repeat region is immunodominant. In the polypeptides of the present invention, the number and positioning of tandem repeats, or repeating units, is selected so as not to mask the immune response to the first and second flanking regions. Preferably, the number of repeats in the polypeptides of the invention is no more than one-half of the number of repeats present in the wild-type protein. More preferably, the number of repeats in the polypeptides of the invention is no more than about one quarter of the number of repeats present in the wild-type protein.

Four species of Plasmodium are known to infect man, the most prevalent being P. falciparum followed by P. vivax and, to a lesser extent, P. malariae and P. ovale.

The central repeat domain of the Plasmodium falciparum sporozoite stage circumsporozoite (CS) protein is comprised of 41 tandemly repeating tetrapeptides, thirty one of which have the amino acid sequence (Asparagine (Asn)-Alanine (Ala)-Asn-Proline (Pro) and four of which have the sequence (Asn-Valine (Val)-Aspartate (Asp)-Pro). On either side the central repeat domain are the flanking regions containing Region I and Region II, two regions of the CS protein nearly identical in amino acid sequence to the corresponding regions of all Plasmodial species such as P. knowlesi (a monkey malaria) CS protein (Dame et al., Science, 225 : 593 (1984)).

Various surface proteins of P. falciparum blood stages also have repeated epitopes, for example, S antigen (Pro-Ala-Lys-Ala-Ser-Gln-Gly-Gly-Leu -Glu-Asp) ; RESA antigen (Glu-Glu-Asn-Val-Glu-His-Asp-Ala) ; FIRA antigen (Val-Thr-Thr-Gln-Glu-Pro) ; and PF-11 antigen (Glu-Glu-Val-Val-Glu-Glu-Val-Val-Pro).

The circumsporozoite protein of the P. vivax contains the repeated epitope (Gly-Asp-Arg-Ala-Asp-Gly-Gln-Pro-Ala) and the circumsporozoite protein of P. malariae contains the repeated epitope (Asn-Asp-Ala-Gly) and (Asn-Ala-Ala-Gly).

The polypeptides of the present invention comprise one or more immunogenic determinants from a first region flanking the central repeat domain of a Plasmodium surface protein, one or more immunogenic determinants from a second region flanking the repeat domain and fewer than all or none of the repeating immunogenic determinants from the central repeat domain.

Immunogenic determinants are amino acid sequences which elicit a B cell or T cell response. Immunogenic determinants generally comprise at least 6 amino acids. Precise identification of immunogenic determinants within a protein can be made by standard techniques involving monoclonal antibody mapping and/or deletion of amino acids followed by activity assay. Preferably, the polypeptides of the invention comprises at least about 15 amino acids from each of the first and second flanking regions ; more preferably, at least about 30 ; and, most preferably, the entire first and second flanking regions, less the signal sequence.

3

In one embodiment, the polypeptides of the present invention comprise at least one but fewer than all immunogenic determinants from a Plasmodium surface protein central repeat domain and one or more immunogenic determinants from regions of the surface protein flanking the repeat domain.

In another embodiment, the polypeptides of the present invention comprise substantially all of the immunogenic determinants from the regions flanking the central repeat domain and are devoid of immunogenic determinants from the central repeat domain or, alternatively, contain at least one but fewer than all immunogenic determinants from the central repeat domain. By "substantially all" is meant that substantially the entire first and second flanking regions, less the signal sequence, are employed ; preferably, no more than about 20 amino acids are lacking from each region, and, more preferably, the entire first and second flanking regions, less the signal sequence, are employed.

Another embodiment of the invention is directed to a polypeptide comprising one or more immunogenic determinants from the first flanking region of a Plasmodium surface protein, one or more immunogenic determinants from the second flanking region of a Plasmodium surface protein, and fewer than all or no repeating immunogenic determinants from the repeat domain therebetween, wherein at least one of said immunogenic determinants from the first flanking region is encoded by the amino acid sequence

Gly-Asp-Asn-Gly-Arg-Glu-Gly-Lys ;
Glu-Lys-Leu-Arg-Lys-Pro-Lys ; or
. Leu-Lys-Gln-Pro-Gly-Asp-Gly-Asn ;
or a functional equivalent thereof.

Yet another embodiment of the invention is directed to a polypeptide comprising one or more immunogenic determinants from the first flanking region of a Plasmodium surface protein, one or more immunogenic determinants from the second flanking region of a Plasmodium surface protein, and fewer than all or no repeating immunogenic determinants from the repeat domain therebetween, wherein at least one of said immunogenic determinants from the second flanking region is encoded by the amino acid sequence

Pro-Asn-Asp-Pro-Asn-Arg-Asn-Val-Asp-Glu-Asn-Ala ; or
Asn-Lys-Pro-Lys-Asp-Glu-Leu-Asp ;
or a functional equivalent thereof.

Still another embodiment of the invention is directed to a polypeptide comprising one or more immunogenic determinants from the first flanking region of a Plasmodium surface protein, one or more immunogenic determinants from the second flanking region of a Plasmodium surface protein, and fewer than all or no repeating immunogenic determinants from the repeat domain therebetween, wherein at least one of said immunogenic determinants from the first flanking region is encoded by the amino acid sequence

Gly-Asp-Asn-Gly-Arg-Glu-Gly-Lys ;
Glu-Lys-Leu-Arg-Lys-Pro-Lys ; or
Leu-Lys-Gln-Pro-Gly-Asp-Gly-Asn ;
and wherein at least one of said immunogenic determinants from the second flanking region is encoded by the amino acid sequence

Pro-Asn-Asp-Pro-Asn-Arg-Asn-Val-Asp-Glu-Asn-Ala ; or
Asn-Lys-Pro-Lys-Asp-Glu-Leu-Asp ;
or a functional equivalent thereof.

A further embodiment of the invention is directed to a polypeptide comprising fewer than all or no repeating immunogenic determinants from the repeat domain of a Plasmodium surface protein and at least one peptide encoded by the amino acid sequence :

Gly-Asp-Asn-Gly-Arg-Glu-Gly-Lys ;
Glu-Lys-Leu-Arg-Lys-Pro-Lys ;
Leu-Lys-Gln-Pro-Gly-Asp-Gly-Asn ;
Pro-Asn-Asp-Pro-Asn-Arg-Asn-Val-Asp-Glu-Asn-Ala ; or
Asn-Lys-Pro-Lys-Asp-Glu-Leu-Asp ;
or a functional equivalent thereof.

A further embodiment of the invention is related to a vaccine for protecting humans against infection against by Plasmodium sporozoites comprising an immunoprotective amount of the polypeptide of the invention and a pharmaceutically acceptable carrier. This invention is also directed to a method of immunizing a human in need of protection against against infection by Plasmodium sporozoites comprising administering an effective, non-toxic amount of such vaccine of to such human.

Preferably, the polypeptide of the present invention ia a hybrid polypeptide, that is, a protein comprised of the genetic or chemical fusion between a portion of a surface protein of Plasmodium and a carrier protein.

More preferred are hybrid polypeptides which include a carrier protein genetically fused to a portion of the Plasmodium falciparum circumsporozoite (CS) protein containing fewer than all or devoid of the repeating tet-

4

rapeptides which comprise the central repeat domain.

Particularly preferred are those hybrid polypeptides in which the chemically fused carrier protein is an immunoenhancing macromolecule, such as, but not limited to killed Bordetella, tetanus toxoid, diptheria toxin and Cholera B toxin.

Also particularly preferred are those hybrid polypeptides in which the genetically fused carrier protein not only enhances immunogencity of the carried polypeptide and which may also enhance expression of the polypeptide by a transformant. Other desirable properties of such carrier proteins include enhancing purification or formulation of the polypeptide. Examples of such carrier proteins include Hepatitis B virus surface antigen (HBsAg), $NSI_{81}$ (81 N-terminal amino acids of influenza virus (A/PR/8/34) non-structural protein 1) (Baez et al., Nucleic Acids Research, 8 : 5845 (1980)) ; R32 ([Asn-Ala-Asn-Pro)$_{15}$- (Asn-Val-Asp-Pro)]$_2$) (Young et al., Science, 228 : 958 (1985)) ; and galK.

Based on the disclosure presented herein, the polypeptides of the invention can be prepared by one of skill in the art using conventional genetic engineering techniques or conventional peptide synthesis techniques.

Specific embodiments of the types of polypeptides of the present invention exemplified herein include :

$NSI_{81}$-RLfΔ9, a fusion polypeptide comprising 81 N-terminal amino acids of influenza virus non-structural protein 1 ($NSI_{81}$) ; the Region I-containing flanking region of the P. falciparum CS protein less the signal sequence (18 N-terminal amino acids) and the Region II-containing flanking region less the first nine N-terminal amino acids thereof (RLfΔ9). Fusion of $NSI_{81}$ to RLfΔ9 is facilitated through a synthetic DNA linker sequence encoding-Asp-His-Met-Leu-Thr-Asp- ;

$NSI_{81}$-RLfAuth, a fusion polypeptide comprising $NSI_{81}$ ; the Region I-containing flanking region of the P. falciparum CS protein less the signal sequence and the entire Region II-containing flanking region (RLfAuth) ;

$NSI_{81}$-(Asn-X-Y-Pro)$_n$-RLfAuth, a fusion polypeptide comprising $NSI_{81}$ ; RLfAuth, and (Asn-X-Y-Pro), wherein X is Ala or Val and Y is Asn or Asp and n is an integer greater than or equal to one and less than or equal to 100, preferably less than 50 ; and, further, wherein the (Asn-X-Y-Pro) is positioned between $NSI_{81}$ and RLfAuth ;

$NSI_{81}$RLfAuth + (Asn-X-Y-Pro)$_n$, a fusion polypeptide comprising $NSI_{81}$ ; RLfAuth ; and (Asn-X-Y-Pro) wherein X and Y are defined as above and n is < 41 ; and, further, wherein (Asn-X-Y-Pro) is positioned between the Region I-containing flanking region of the P. falciparum CS protein and the Region II-containing flanking region, that is, the region formerly occupied by the central repeat domain ; and

The following peptides of the invention which were identified as sporozoite neutralizing epitopes of P. falciparum :

Gly-Asp-Asn-Gly-Arg-Glu-Gly-Lys

Glu-Lys-Leu-Arg-Lys-Pro-Lys

Leu-Lys-Gln-Pro-Gly-Asp-Gly-Asn

Pro-Asn-Asp-Pro-Asn-Arg-Asn-Val-Asp-Glu-Asn-Ala

Asn-Lys-Pro-Lys-Asp-Glu-Leu-Asp

Such polypeptides are, however, illustrative only. Based on the disclosure provided herein, one skilled in the art will know how to construct and test other polypeptides within the scope of the invention, for example, polypeptides comprising sequences from surface proteins, including circumsporozoite protein of the various malaria parasites other than P. falciparum, polypeptides comprising more or fewer amino acids from the surface proteins, polypeptides which are chemically modified, and polypeptides which are fused to other or additional amino acid or protein sequences. Such polypeptides are readily constructed by standard techniques of genetic engineering and/or protein synthesis and can be tested in animal models substantially as described hereinbelow. For example, a protein of the invention may comprise amino acid sequences from surface protein flanking regions, such as substantially the entire circumsporozoite protein devoid of the central repeat domain, fused to the surface antigen of Hepatitis B Virus (HBsAg) in a fusion protein which can form hybrid HBsAg particles, as described in European patent application publication number EP 278,940 published August 17, 1988.

A genetic coding sequence for surface protein flanking regions, tetrapeptides, synthetic DNA linker sequences, and carrier proteins can be easily obtained by one skilled in the art using known techniques. These include synthesis and, preferably, by reverse transcription of messenger RNA or by direct cloning of intact genes from genomic DNA as well as generation of specific sequences by conventional polymerase chain reaction (PCR) technology. Reverse transcription of P. falciparum messenger RNA is described in Ellis et al., Nature 302 : 536 (1963). Direct cloning of intact genes from P. falciparum genomic DNA is described in Dame et al., (cited above). Cloning and expression of repeat-containing polypeptides is described in published European Patent Application No. 86870014.7, filed February 3, 1990, the disclosure of which is incorporated herein by reference.

Having cloned all or a portion of Plasmodium DNA, fragments thereof encoding all or a portion of the surface protein can be prepared by known techniques.

Techniques for synthesizing DNA are well known and may be accomplished using commercially available DNA synthesizers.

Coding sequences for polypeptides may be inserted into E. coli expression vectors, many of which are known and readily available. In carrying out the present invention in E. coli, a DNA sequence which encodes the polypeptide of the present invention is operatively linked to a regulatory element within a DNA vector for transformation in E. coli. Numerous gram negative bacterial expression vectors comprising such regulatory elements are available. The regulatory element comprises a promoter which effect RNA polymerase binding and transcription. Regulatable, that is, inducible or derepressable, promoters are preferred. A variety of useful promoters and available for expression of heterologous polypeptides in E. coli. These include the trp promoter and the lambda PL promoter (eg. U.S. Patent No. 4.578.355 and Courtney et al., Nature 313 : 149 (1985). As described in more detail below, it has been found that coding sequences encoding the polypeptides of the present invention are particularly well expressed by the E. coli expression vector pMG-1. Derivatives of pMG-1, encoding carrier proteins other than NSI$_{81}$, for example, R32 and galK, may also be used to advantage.

In carrying out the present invention in Streptomyces, a DNA coding sequence which encodes the polypeptide of the present invention is operatively linked to a regulatory element within a DNA vector for transformation of Streptomyces. The regulatory element comprises a promoter which effects RNA polymerase binding and transcription. Regulatable, i.e., inducible or derepressible, promoters are preferred. A variety of useful promoters are available for exprssion of heterologous polypeptides in Streptomyces. Examples include the galactose-inducible promoter of the Streptomyces galactose operon (Fornwald, et al., Proc Natl. Acad. Sci. USA 84 : 2130 (1987)), the constitutive promoter of the S. lividans β-galactosidase gene (Eckhardt, et al. J. Bacteriol. 169 : 4249 (1987) ; Brawner, et al., U.S. Patent 4,717,666) and the S. longisporus trypsin inhibitor gene (European Patent Application No. 87 307 260.7), or a temporally regulated promoter such as that reported in M. echinosporsa (Baum, et al., J. Bacteriol 170 : 71 (1988)). Regions for transcription termination in Streptomyces are derived from the 3' end of several Streptomyces genes, for example the termination signal at the end of the Streptomyces galactose operon or that found at the end of the S. fradiae neomycin phosphotransferase gene (Thompson and Gray, Proc. Natl. Acad. Sci USA 80 : 5190 (1983)). Sequences for protein export in Streptomyces include those isolated from the S. lividans β-galactosidase gene, the S. lividans LEP-10 gene (European Patent Application No. 87 307 260.7) and the S. longisporus trypsin inhibitor gene.

The gene encoding the polypeptide of the present invention is incorporated into a larger DNA molecule which comprises a genetic selection marker system. The selection marker system can be any of a number of known marker systems such that the marker gene confers a selectable new phenotype on the transformed cell. Examples include Streptomyces drug resistance genes such as thiostrepton resistance ribosomal methylase (Thompson, et al., Gene 20 : 51 (1982)), neomycin phosphotransferase (Thompson, et al., supra) and erthromycin resistance ribosomal methylase (Thompson, et al., supra). The DNA molecule may also contain a sequence for autonomous replication in Streptomyces, such as the pIJ101 derivatives (Keiser, et al., Mol. Gen. Genet. 185 : 223 (1982)) or an SLP1 derived vector (Bibb, et al., Mol. Gen. Genet. 184 : 230 (1981)). The DNA molecule may also contain a marker which permits gene amplification. Such markers which serve to amplify gene copy number in Streptomyces include the gene for spectinomycin resistance (Hornemann, et al., J. Bacteriol 169 : 2360 (1987)) and arginine auxotrophy (Altenbuchner, et al., Mol. Gen. Genet. 195 : 134 (1984)).

In carrying out the present invention in yeast, a DNA coding sequence which encodes the polypeptides of the present invention is operatively linked to a regulatory element within a DNA vector for transformation of yeast. Any yeast host for which transformation, cloning and expession systems are available can be used. Particular examples include yeasts of the genera Hansenula, Pichia, Kluveromyces, Schizosaccharomyces, Candida and Saccharomyces. The preferred yeast host is Saccharomyces cerevisiae.

The regulatory element comprises a promoter which effects RNA polymerase binding and transcription. Regulatable, i.e., inducible or derepressible, promoters are preferred. A variety of useful promoters are available for expression of heterologous polypeptides in yeast. These include the copper inducible metallothionine gene (CUP1) promoter and the constitutive promoter of the glycolytic genes glyceraldehye-3 phosphate dehydrogenase (TDH3) and alcohol dehydrogenase (ADH). Regions for transcriptional termination in yeast are derived from the 3' end of any of several yeast genes, for example the gene for iso-1-cytochrome C (CYC1)

The gene encoding the polypeptide of the present invention is incorporated into a larger DNA molecule which comprises a genetic selection marker system. The selection marker system can be any of a number of known marker systems, such that the marker gene confers a selectable new phenotype on the transformed cell. Examples include yeast genes for biosynthetic enzymes such as phospho-ribosyl anthranilate isomerase (TRP1) or orotidine-5'-phosphate decarboxylase (URA3) or heterologous drug resistance genes such as G418 resistance or benomyl anthranilate isomerase (TRP1) or benomyl resistance (BEN1). The DNA molecule may also contain a sequence for autonomous replication in yeast, such as the yeast 2-micron-circle ori region or a

6

chromosomal autonomous replication region (ARS), such as ARS1, and a yeast centromere (CEN), such as CEN3, to allow for autonomous replication of the plasmid.

Still other expression systems are known and readily available. For example, a variety of insect cells and expression systems therefor are available for expression of heterologous proteins, such as a baculovirus expression system for use in expressing heterologous proteins in Lepidoptera cells. Where necessary to effect expression in eukaryotic expression systems, it may be necessary to delete the carboxy terminal anchor region of the surface protein. By way of example, deletion of amino acids 392-412, the sequence encompassing the P. falciparum carboxy terminal anchor region may be required

Another exemplary expression system relates to a Salmonella bacterial strain transformed with a selected heterologous gene operatively linked to an E. coli promoter sequence, the transformant being capable of constitutively expressing the product of the heterologous gene.

The polypeptides so expressed are isolated and purified from the producing cell culture using standard protein isolation techniques, many of which are well known in the art. An exemplary, useful purification scheme comprises (1) the disruption of the bacterial cells, (2) clarification of cellular debris, (3) separation of the polypeptides of the present invention from other polypeptides present in the clarified cell extract, and (4) final purification to remove residual contaminants, including residual polypeptides, carbohydrates, nucleic acids, lipopolysaccharides and endotoxins.

In the vaccine of the invention, an aqueous solution of the polypeptide, preferably buffered at physiological pH, can be used directly. Alternatively, the polypeptides can be admixed or absorbed with any of a number of known adjuvants. Such adjuvants include, for example, aluminum hydroxide, muramyl dipeptide and saponons such as Quil A. As a further example, the polypeptide may be encapsulated within microparticles such as liposomes. In yet another alternative, the polypeptides of the present invention may be conjugated (fused chemically) via conventional techniques to an immunoenhancing macromolecule, such as killed Bordetella or a tetanus toxoid, diptheria toxin or Cholera B toxin.

Vaccine preparations are generally described in New Trends and Developments in Vaccines, Voller et al., Eds., University Park Press, Baltimore, MD, USA (1978). Encapsulation within liposomes is described, for example, in U.S. Patent No. 4,235,877 to Fullerton. Conjugation of proteins to macromolecules is disclosed in U.S. Patent No. 4,372,945 to Likhite and U.S. Patent No. 4,474,757 to Armor et al. Use of Quil A is disclosed, for example, by Dalsgaard et al., Acta Vet. Scand., 18 : 349 (1977).

The amount of polypeptide present in each vaccine dose is that amount which induces an immunoprotective response without significant, adverse side effects. Such amounts will vary according to the specific polypeptide employed and whether or not the vaccine is adjuvanted. Generally, it is expected that each dose will comprise 1 to 1000 ug. of polypeptide, preferably 10 to 200 ug. An optimal amount for a particular vaccine can be ascertained by standard studies involving observation of antibody titers and other responses in subjects. Following an initial vaccination, subjects will preferably receive a boost in about four weeks, followed by additional boosts every six months for as long as the risk of infection exists.

Intramuscular, subcutaneous or intravenous administrations are generally preferred, although in some cases, other routes may be useful. For example, where recombinant Salmonella are employed, the preferred route of administration may be oral.

The following Examples are illustrative, and not limiting, of the invention. The CS protein coding sequence was supplied by James Weber, Walter Reed Army Institute for Research, as a 2337 base pair EcoR I fragment of λmPF1 (Dame et al., Science 225 : 593 (1984)) in the EcoR I site of pUC8, a standard E. coli cloning vector (available, for example, from Bethesda Research Laboratories, Inc., Gaithersburg, MD). The resulting pUC8 derivative is referred to as pUC8 clone 1.

EXAMPLE 1

Construction of pNSI$_{81}$RLfΔ9

Briefly summarized, construction of pNSI$_{81}$RLfΔ9 was completed as follows. A first aliquot of pCSP (described below), an E. coli expression vector containing a 1216 base pair fragment encoding all but the first 18 amino acids of the P. falciparum circumsporozoite (CS) protein, was digested with restriction endonuclease Fok I, end-filled (Klenow Fragment) and digested with restriction endonuclease BamH I. The resulting 318 base pair fragment, encoding amino acids 19 (Leu) to 123 (Pro) of the CS protein, was recovered by electroelution.

A second aliquot of pCSP was digested with restriction endonuclease TthIII I, end-filled and digested with restriction endonuclease Sal I. The resulting 655 base pair fragment, encoding amino acids 297 (Gly) to 412 (Asn) of the CS protein, was recovered by electroelution. (This sequence is lacking 9 N-terminal amino acids, Nos. 288 (Pro) to 296 (Gln), of the Region II-containing flanking region.)

The 318 base pair and 655 base pair CS protein gene fragments were ligated into E. coli expression vector pUC18 (described below) previously digested with restriction endonuclease BamH I and Sal I. The resulting vector was named pUCRLfΔ9.

pUCRLfΔ9 was digested with restriction endonuclease BamH I, end-filled, and digested with restriction endonuclease Sal I. The resulting 1035 base pair fragment, encoding CS protein amino acids 19 to 123 and 297 to 412, was recovered by electroelution.

Expression vector pMG-1 (described below), containing a DNA fragment encoding N-terminal amino acids 1 (Met) to 81 (Met) of influenza virus non-structural protein 1 and a synthetic DNA linker sequence, was digested with restriction endonuclease EcoR V and Xho I. The 1035 base pair fragment, previously isolated from pUCRLfΔ9, was then ligated into pMG-1. The resulting expression vector, pNSl$_{81}$RLfΔ9 encodes a protein having the following sequence :

NSl$_{81}$-Asp-His-Met-Leu-Thr-Asp-Pro-CS$_{19-123}$-CS$_{297-412}$.

Construction of pNSl$_{81}$RLfΔ9 is detailed below.

A. Construction of pCSP

Purified pUC8 clone 1 plasmid DNA (40 µg.) was digested with restriction endonucleases Stu I and Rsa I (100 units of each enzyme) in 400 ul. of medium buffer (comprising 50 mM Tris, 50 mM NaCl, 1 mM dithiothreitol (DTT), and 10 mM MgC1$_2$, having a pH of 7.5) for 1.5 hours at 37°C. The resulting 1216 base pair fragment, encoding all but the first 18 amino acids (believed to encode the CS protein signal sequence) of the circumsporozoite (CS) protein, was isolated by electrophoresis on a 6% polyacrylamide gel (PAGE) and recovered by electroelution.

Ten micrograms of expression vector pAS1 (ATCC 39262, more fully described in U.S. Patent No. 4,578,355 to M. Rosenberg) was digested with restriction endonuclease BamH I (25 units) in 200 µl. medium buffer (described above) for 1.5 hours at 37°C. The cut plasmid was then treated for 15 minutes at 25°C with DNA Polymerase I, Large Fragment (5 units of Klenow Fragment in 20 mM Tris-HC1, pH 7.5, 7 mM MgC1$_2$, 60 mM NaC1, 6 mM 2-mercaptoethanol and 0.25 mM of each of the four deoxynucleotide triphosphates to end-fill the BamH I site).

The circumsporozoite protein gene fragment (1 µg.) was then ligated into this vector (100 ng.) in 30 µl. ligase buffer (comprising 50 mM Tris, 1 mM DTT, 10 mM MgC1$_2$, 100 µM rATP, having a pH of 7.5) with one unit of T4-DNA ligase for 16 hours at 4°C.

The ligation mixture was transformed into E. coli strain MM294Cl +. Ampicillin resistant colonies were obtained and screened for insertion of the CS gene fragment into pAS1. A plasmid with the correct construction (pCSP) was identified.

B. Construction of pUCRLfW9

Purified pCSP plasmid DNA (100 µg.) was digested with restriction endonuclease Fok I (100 units) in 400 µl. of medium buffer (described above) for 3 hours at 37°C. Subsequently, the plasmid was treated with DNA Polymerase I, Large Fragment (described above) to end-fill the Fok I site. The plasmid was next digested with restriction endonuclease BamH I (100 units) in 400 µl. of medium buffer (described above) for 3 hours at 37°C. The resulting 318 base pair fragment, encoding amino acids 19-123 of the CS protein, was isolated by electrophoresis on a 6% polyacrylamide gel (PAGE) and recovered by electroelution.

An additional aliquot (100 µg.) of pCSP was digested with restriction endonuclease TthIII I (100 units) in 400 µl. of medium buffer (described above) for 3 hours at 65°C. Subsequently, the plasmid was treated with DNA Polymerase I, Large Fragment (described above) to fill in the TthIII I site. The plasmid was next digested with restriction endonuclease Sal I (100 units) in 400 µl. of medium buffer for 3 hours at 37°C and the resulting 655 base pair fragment, encoding amino acids 297-412 of the CS protein, was isolated by electrophoresis on a 6% polyacrylamide gel and recovered by electroelution.

Ten micrograms of expression vector pUC18 (Yanish-Perron et al., Gene, 33 : 103 (1985)), a standard E coli cloning vector, (available, for example, from Bethesda Research Laboratories, Inc., Gaithersburg, MD) was digested with restriction endonuclease BamH I and Sal I (20 units each) in 200 µl. of medium buffer (described above) for 2 hours at 37°C. The 318 base pair BamH I end-filled/Fok I fragment (1 µg.) and the 655 base pair TthIII I end-filled/Sal I fragment (1 µg.) was then ligated into pUC18 in 30 µl. ligase buffer (described above) with one unit of T4-DNA ligase for 16 hours at 4°C. A plasmid with the correct construction (pUCRLfΔ9) was identified.

C. Construction of pMG-1

Ten micrograms of expression vector pMG27N- (M.Gross et al., Mol. Cell. Biol., 5 : 1015 (1985)) was digested with restriction endonucleases BamH I and Sac I (50 units of each) in 200 µl. medium buffer (described above) for 3 hrs. at 37°C.

Ten micrograms of expression vector pAPR801 (Young et al., Proc. Natl. Acad. Sci. U.S.A., 80 : 6105 (1983)) containing the influenza virus (A/PR/8/34) non-structural protein 1 (NS1) coding region (Baez, et al., Nucleic Acids Research, 8 : 5845 (1980)) was digested with restriction endonucleases NcoI and BamH I (20 units each) in 200 µl. of high buffer (50 mM Tris-HCl, 1 mM DTT, 10 mM MgCl$_2$, and 100 mM NaC1, pH of 7.5) for 2 hours at 37°C. The resulting 230 base pair fragment, encoding the first 81 N-terminal amino acids of NS1, was isolated by electrophoresis on a 6% polyacrylamide gel (PAGE) and recovered by electroelution.

Forty nanograms of the BamH I/Sac I-cut pMG27N-(described above) was ligated with 80 ng. of the 230 base pair Nco I/BamH I NSI$_{81}$-encoding fragment and 80 ng. of a synthetic linker having the following sequence:

$$5\,'\,CATGGATCATATGTTAACAGATATCAAGGCCTGACTGACTGAGAGCT\ \ 3\,'$$
$$3\,'\quad\quad CTAGTATACAATTGTCTATAGTTCCGGACTGACTGACTC\quad\quad\quad 5\,'$$

The resulting plasmid, pMG-1, was identified with the BamH I site of the NSI$_{81}$ encoding sequence ligated to the BamH I site of pMG27N- ; the Nco I site of the NSI$_{81}$ encoding sequence ligated to the Nco I site of the synthetic linker ; and the Sac I site of the synthetic linker ligated to the Sac I site of pMG27N-. This vector introduces unique restriction sites to facilitate insertion of DNA fragments in any of three reading frames, results in the insertion of TGA termination codons in all three reading frames downstream of the ATG initiation codon of the cII ribosome binding site and, when expressed, results in NSI$_{81}$ fusion proteins from all three reading frames. Digestion of pMG-1 with restriction endonuclease Nde I and subsequent ligation of the vector as described above results in the expression of a non-fusion protein (that is, not fused to NSI$_{81}$).

D. Construction of pNSI$_{81}$RLfΔ9

Expression vector pUCRLf (100 µg.) was digested with restriction endonuclease BamH I (100 units) in 400 µl. high buffer (described above) for 3 hours at 37°C. The cut plasmid was subsequently treated with DNA Polymerase I, Large Fragment (described above) to end-fill the BamH I site. The plasmid was next digested with restriction endonuclease Sal I (20 units) in 400 µl. medium buffer (described above) for 3 hours at 37°C. The resulting 1035 base pair fragment was isolated by electrophoresis on a 6% polyacrylamide gel (PAGE) and recovered by electroelution.

Expression vector pMG-1 (10 µg.) was digested with restriction endonucleases EcoR V and Xho I (25 units of each) in 400 µl. of medium buffer (described above) for 3 hours at 37°C. The 1035 base base pair BamH I end-filled/ Sal I (400 µg.) fragment from pUCRLf was then ligated into this vector (100 ng.) in 30 ul. of ligase buffer (described above) with one unit of T4-DNA ligase for 16 hours at 4°C.

The ligation mixture was transformed into E. coli strain MM294C1 +. Ampicillin resistant colonies were obtained and screened for clones containing the properly oriented inserted gene. A plasmid with the correct construction (pNSI$_{81}$RLfΔ9) was identified, transformed in E. coli strain AR58 (cIts857) and tested for expression of the circumsporozoite protein gene product devoid of the first 18 N-terminal amino acids (CS$_{1-18}$), the central repeat domain, and 9 N-terminal amino acids (CS$_{248-296}$) of the Region II-containing flanking region (RLfΔ9), fused, via 6 amino acids (Asp-His-Met-Leu-Thr-Asp) derived from the synthetic linker ligated into the pMG-1 expression vector, to 81 N-terminal amino acids of the influenza non-structural protein 1, NSI$_{81}$. NSI$_{81}$RLfΔ9 has the following sequence :

NSI$_{81}$-Asp-His-Met-Leu-Thr-Asp-Pro-CS$_{19-123}$-CS$_{297-412}$.

The proline separating the Asp (from the C terminus of the synthetic linker) from RLfΔ9 (CS$_{19-123}$-CS$_{297-412}$) is an artifact of the filled-in BamH I site of the BamH I/Fok I fragment of pCSP.

Cells were grown in Luria-Bertani Broth (LB) at 32°C to an absorbance at 650 nm (A$_{650}$) of 0.6 and temperature induced at 42°C for 3 hours to turn on transcription of the PL promoter of the expression plasmid and subsequent translation of the NSI$_{81}$ CS protein derivative. Cells were sampled in 1 ml. aliquots, pelleted, resuspended in lysis buffer (10 mM Tris-HCl, pH 7.8, 25% (vol/vol) glycerol, 2% 2-mercaptoethanol, 2% sodium dodecyl sulfate (SDS), 0.1% bromophenyl blue) and incubated in a 105°C heating block for 5 minutes.

Proteins were separated by SDS-PAGE (13% acrylamide, 30 : 0.8 acrylamide : bis-acrylamide ratio). Proteins were transferred to nitrocellulose and the NSI$_{81}$RLfΔ9 protein produced in E. coli was detected by Western Blot analysis using polyclonal antibodies reactive with a domain of the CS protein called Region I (Dame et al, Science 225 : 593 (1984)) as well as polyclonal antibodies reactive with NSI$_{81}$ protein. The E. coli produced NSI$_{81}$RLfΔ9 protein was also shown to be non-reactive with a pool of 5 monoclonal antibodies directed to the tetrapeptide repeat domain of the P. falciparum CS protein.

EXAMPLE 2

Construction of pNSI₈₁RLfAuth

E. coli expression vector pCSP (described above) was digested with restriction endonuclease BamH I and Fok I in medium buffer. The resulting DNA fragment, encoding the Region I-containing flanking region, less the first 18 N-terminal amino acids, was recovered by electroelution.

A second aliquot of pCSP was digested with restriction endonuclease Tthlll I and Sal I in medium buffer. The resulting DNA fragment, encoding the Region II-containing flanking region, less the first 9 N-terminal amino acids, was recovered by electroelution.

E. coli expression vector pUC18 (described above) was digested with restriction endonuclease BamH I and Sal I in medium buffer.

To restore the 9 N-terminal amino acids ($CS_{288-296}$) of the Region II-containing region of the C-terminal flank of the central repeat domain of the CS protein, which amino acids were lost in the digestion of pCSP with restriction endonuclease Tthlll I, a synthetic DNA fragment containing a Fok I end and a Tthlll I end, and having the following sequence, was prepared :

```
              AspProGlyAsnLysAsnAsnGlnGlyAsnGlyGln
    FokI  5' ATCCCGGGAATAAAAACAACCAAGGTAATGGACA  3' Tth 111 I
          3'         GCCCTTATTTTTGTTGGTTCCATTACCTGTT  5'
```

The BamH I/Fok I fragment, Tthlll I/Sal I fragment and the synthetic fragment were ligated into the BamH I/ Sal I digested pUC18.

The resulting plasmid, pUCRLfAuth, was digested with restriction endonuclease BamH I in medium buffer, end-filled, and digested with restriction endonuclease Sal I. The resulting DNA fragment, encoding authentic circumsporozoite protein lacking the first 18 N-terminal amino acids and the central repeat domain, was recovered by electroelution.

The isolated BamH I end-filled/Sal I fragment was then ligated into the NSI₈₁-encoding E. coli expression vector, pMG-1 (described above), which had previously been digested with restriction endonuclease EcoR V and Xho I. The resulting expression vector, pNSI₈₁RLfAuth, expresses a protein having the sequence :
NSI₈₁-Asp-His-Met-Leu-Thr-Asp-Pro-$CS_{19-123}$-Gly-$CS_{288-412}$
(The glycine separating the Region I and Region II-containing CS flanking regions ($CS_{19-123}$ and $CS_{288-412}$) is an artifact of the synthetic Fok I/Tthlll I DNA linker sequence.

The complete nucleotide and amino acid sequence for NSI₈₁RLfAuth is available in published European Patent Application Number 90304720.7, filed May 1, 1990, the entire disclosure of which is incorporated by reference.

EXAMPLE 3

Construction of pNSI₈₁RLfAuth + (NANP)₂

pNSI₈₁RLfAuth (described above) was digested with restriction endonuclease Sma I in medium buffer (described above and containing KCl) at 25°C for 3 hours.

A synthetic DNA linker, having the sequence :

```
          AsnAlaAsnProAsnAlaAsnPro
     5' AACGCAAACCCAAATGCAAACCCC  3'
     3' TTGCCTTTGGGTTTACGTTTGGGG  5'
```

was ligated into the Sma I-digested pNSI₈₁RLfAuth. The synthetic DNA linker encodes (NANP)₂ (single letter symbols designating amino acids, N = asparagine (Asn) ; A = alanine (Ala) ; and P = proline (Pro)), the tetrapeptide comprising the so-called consensus sequence of the immunodominant repeat domain of the CS protein. Digestion of pNSI₈₁RLfAuth with restriction endonuclease Sma I permits ligation of any number of repeating tetrapeptides encoded by a synthetic DNA linker into that region of the CS protein formerly occupied by the immunodominant repeat domain. Additional tetrapeptide-encoding DNA fragments may be ligated into the vector in this manner. The resulting plasmid, pNSI₈₁RLfAuth + (NANP)₂ encodes a protein having the sequence :

NSI$_{81}$-Asp-His-Met-Leu-Thr-Asp-Pro-CS$_{19-123}$-(NANP)$_2$ -Gly-CS$_{288-412}$

## EXAMPLE 4

Construction of pNSI$_{81}$(NANP)$_4$RLfAuth

Expression vector pUCRLfAuth (described above) was digested with restriction endonuclease BamH I.

A synthetic DNA fragment, encoding (NANP)$_4$ was ligated into the BamH I digested pUCRLf. The synthetic DNA fragment had the following sequence :

```
        ProAsnAlaAsnProAsnAlaAsnProAsnAlaAsnProAsnAlaAsnPro
5 ' GATCCCAATGCAAACCCAAATGCAAACCCAAACGCTAACCCCAACGCTAACCCC 3 '
3 '     GGTTACGTTTGGGTTTACGTTTGGGTTTGCGATTGGGGTTGCGATTGGGGCTAG 5 '
```

The resulting plasmid was named pUC18(NANP)$_4$RLfAuth

Expression vector pUC18(NANP)$_4$RLfAuth was digested with restriction endonuclease BamH I, end-filled (Klenow Fragment), digested with restriction endonuclease Sal I, and the resulting DNA base pair fragment covered by electroelution.

The BamH I end-filled/Sal I fragment was ligated into the NSI$_{81}$-encoding expression vector pMG1 (described above), previously digested with restriction endonuclease EcoR V and Xho I. The resulting plasmid was named pNSI$_{81}$(NANP)$_4$RLfAuth and encodes a protein wherein repeating tetrapeptides encoded by the synthetic DNA fragment are inserted between amino acid 81 (Met) of NSI$_{81}$ and N-terminal Asp of the Nco I/Sac I synthetic DNA linker :

NSI$_{81}$-(NANP)$_4$-Asp-His-Met-Leu-Thr-Asp-Pro-CS$_{19-123}$ -Gly-CS$_{288-412}$

## EXAMPLE 5

Construction of pNSI$_{81}$(NVDP)$_4$RLfAuth

Construction of pNSI$_{81}$(NVDP)$_4$RLfAuth was the same as that described above for pNSI$_{81}$(NANP)$_4$RLfAuth except that the synthetic DNA linker, encoding (NVDP)$_4$ (the variant tetrapeptide sequence of the CS protein central repeat domain), had the following sequence :

```
              AsnValAspProAsnValAspProAsnValAspProAsnVal
5 ' GATCCCAATGTAGACCCCAACGTTGATCCGAACGTAGACCCGAATGTA 3 '
3 '     GGTTACATCTGGGGTTGCAACTAGGCTTGCATCTGGGCTTACAT 5 '
```

The resulting plasmid encodes a protein having the sequence :
NSI$_{81}$(NVDP)$_4$-Asp-His-Met-Leu-Thr-Asp-Pro-CS$_{19-123}$-GlyCS$_{288-412}$

## EXAMPLE 6

Isolation of NSI$_{81}$RLfΔ9 from E. coli

Following the induction of synthesis of NSI$_{81}$RLfΔ9 in a temperature sensitive lambda lysogen (cl857), the bacterial cells were collected by centrifugation and the resulting pellet frozen at -70°C. Approximately 12 g. of the concentrated and frozen cells were thawed by dilution in 120 ml. of a lysis buffer solution (pH 8) containing 50 mM Tris(hydroxymethyl) aminomethane (TRIS), 10 mM ethylenediaminetetraacetic acid (EDTA), 5% glycerol and 10 mM dithiothreitol (DTT). Lysozyme (Sigma Chemical Co., St. Louis, MO) was added to a final concentration of 0.2 mg./ml. of diluted cells and the solution stirred at 4°C for 30 minutes. The cells were sonicated using a Branson sonicator until the solution appeared liquified. A 10% deoxycholate solution was added to a final concentration of 0.1% (v/v) and the solution was centrifuged at 15,600 × G for 30 minutes at 4°C in a Sorvall RC 5B centrifuge (Dupont).

The supernatant was discarded and the remaining protein-containing pellet was suspended in 100 ml. of a buffer solution (pH 10) containing 50 mM glycine, 2 mM EDTA, and 5% glycerol. The suspension was sonicated as described above and Triton® X-100 (Sigma) added to a final concentration of 1% (v/v). The sonicated solution was stirred at 4°C for 30 minutes and centrifuged as described above.

Urea was added to the protein-containing supernatant to a final urea concentration of 8 M and the sample titrated to pH 5.5 with a 50% solution of acetic acid. The sample was chromatographed on a 25 ml. column of QAE Sepharose® Fast Flow (Pharmacia) previously equilibrated in a buffer solution (pH 5.5) containing 20 mM sodium acetate, 1% Triton X-100, and 8 M urea, at a flow rate of 300 cm./hr. The protein was in the unbound fraction and applied to a 10 ml. column of SP Sepharose® Fast Flow (Pharmacia) previously equilibrated in a buffer solution (pH 5.5) containing 20 mM sodium acetate and 8M urea, at a flow rate of 120 cm./hr. The effluent was monitored for absorbance at 280 nm. The protein was eluted from this column with a buffer (pH 8) containing 100 mM Tris and 8 M urea.

SDS-PAGE of the resulting product revealed a major band with an apparent Mr of 40,000 kD, and of approximately 80% purity. The purification process yielded 12 mg. of protein containing approximately 14 endotoxin units/mg. of protein.

## EXAMPLE 7

Isolation of $NSI_{81}RLf\Delta9$ from E. coli

Following the induction of synthesis of $NSI_{81}RLf\Delta9$ in a temperature sensitive lambda lysogen, the bacterial cells were collected by centrifugation and the resulting pellet frozen at -70°C. Approximately 636 g. of the concentrated and frozen cells were thawed by dilution in 2200 ml. of a buffer solution (pH 8.0) containing 60 mM Tris, 12 mM EDTA, 6% glycerol and 12 mM dithiothreitol (DTT). The thawed cells were passed through a Manton Gaulin homogenizer two times at 6000-7000 psi. A 10% deoxycholate solution was added to a final concentration of 0.1% (v/v), the lysate stirred at 4°C for 30 minutes and centrifuged at 10,000 × G in a Sorvall RC 5B centrifuge (Dupont) at 4°C for 60 minutes.

The pellet was discarded and 25 µl. of 1050 or 2100 Biocryl bead mixture (Supelco) was added to each ml. of the protein-containing supernatant. The solution was stirred and centrifuged as described above.

The pellet was discarded and solid ammonium sulfate was added over a five minute period to the remaining protein-containing supernatant to 20% saturation.

The sample was stirred and centrifuged as described above. The pellet was suspended in 400 ml. of a buffer solution (pH 5.5) containing 20 mM sodium acetate, 1% Triton® X-100 and 8 M urea. This suspension was centrifuged and the supernatant was dialyzed against a buffer solution (pH 8.0) containing 100 mM Tris and 8 M urea. The retentate was adjusted to pH 5.5 and chromatographed on a 100 ml. column of SP Sepharose®, Fast Flow (Pharmacia) previously equilibrated with a buffer solution (pH 5.5) containing 20 mM sodium acetate, 1% Triton® X-100 and 8 M urea at a flow rate of 10 ml./min. The column was washed with equilibration buffer, followed by a buffer containing 0.1 M Tris and 8 M urea at pH 8. The column was eluted with a 500 ml. linear gradient of 0.0 to 0.5 M sodium chloride prepared in a buffer (pH 8) containing 0.1 M Tris and 8 M urea.

The protein eluted at 0.3 M sodium chloride. The fractions containing the product were pooled and concentrated in an Amicon stirred cell using a YM 10 membrane and dialyzed against a buffer solution (pH 8.0) containing 20 mM Tris, followed by dialysis against phosphate buffered saline (pH 7.0).

SDS-PAGE analysis of the resulting product revealed a major band with an apparent Mr of 40,000 kD and a series of minor components. The purification process yielded 25 mg. of protein containing 144 endotoxin units/mg. protein.

## EXAMPLE 8

Isolation of R16CSP from E. coli

R16 CSP, expressed in E. coli, was prepared by ligating an Xho II fragment isolated from pUC8 clone 1 (described above) into pCSP (described above) previously digested with restriction endonuclease BamH I. R16CSP, used as an ELISA capture antigen in Example 9 below, was purified as follows.

Following the induction of synthesis of R16CSP in E. coli, the bacterial cells were collected by centrifugation and the resulting pellet frozen at -20°C. Approximately 373 g. of the concentrated and frozen cells was thawed in 1.43 1. of buffer (pH 3.0) containing 50 mM Tris, 10 mM EDTA, 5% glycerol, and 10 mM dithiothreitol. A 10% deoxycholate (w/v) solution was added to a final concentration of 0.1% (v/v), after which the cells were twice passed through a Manton-Gaulin homogenizer at 7,000 psi. A 10% solution of polyethyleneimine (BRL) in 0.5 M Tris, pH 8.0 buffer (w/v) was added to the homogenate to a final concentration of 0.5%. The solution was stirred at 4°C for 1 hour and centrifuged at 13,000 × g in a Sorvall RC 2B centrifuge (Dupont) at 4°C for 45 minutes.

12

The pellet was discarded and solid ammonium sulfate was added to the supernatant to a saturation of 35% over 5 minutes. The solution was stirred and centrifuged as previously described.

The pellet was suspended in a buffer (pH 8.0) containing 300 ml. of 20 mM Tris, and 10 mM EDTA. A solution containing 300 ml. of 8 M urea, 2.7 l. of 10 mM sodium acetate, and 4 M urea were added and the pH was adjusted to 4.0 immediately. The sample was centrifuged as described previously.

The supernatant was applied to a 50 ml. column of SP-Sepharose®, Fast Flow (Pharmacia) equilibrated in a buffer (pH 4.0) containing 20 mM sodium acetate and 4 M urea. The column was washed with a buffer (pH 5.0) containing 20 mM sodium acetate and eluted with a 250 ml. linear gradient containing 0.0 to 1.0 M sodium chloride in wash buffer. The product eluted at approximately 0.3 M sodium chloride.

A 50 ml. aliquot of the ion exchange product was adjusted to pH 2.3 with 10% trifluoroacetic acid (TFA)(v/v) and chromatographed on a C4 reverse phase column (Vydac, 1 X 25 cm.) equilibrated in 0.1% TFA. A linear gradient of 0 to 60% acetonitrile (ACN) in 0.1% TFA was run over 45 minutes and the product eluted at approximately 60% ACN. The reverse phase product was neutralized by the addition of 25 ul./ml. of 1 M ammonium bicarbonate.

Approximately 45 ml. of the reversed phase product was dialyzed against a buffer (pH 5.0) containing 2 M guanidine hydrochloride, and concentrated to 20 ml. on a YM 30 membrane (Amicon). A 10 ml. aliquot was chromatographed in a 2.5 X 50 cm. column of Superose® 12 (Pharmacia) equilibrated in a buffer (pH 5.0) containing 2.0 M guanidine hydrochloride. The protein which eluted at an apparent Mr of 358kD was dialyzed against a buffer (pH 4.5) containing 20 mM sodium acetate.

Coomassie stained SDS-PAGE of the Superose product revealed two major bands with an apparent Mr of 72kD and 70kD. Amino acid analysis and N-terminal sequencing of the final product were within 15% of the expected value.


## EXAMPLE 9

Antibody Responses of Mice to $NSI_{81}RLf\Delta9$

To evaluate its immunogenicity, purified $NSI_{81}RLf\Delta9$ antigen was inoculated into three strains of mice, C57BL/6 (H-2$^b$) ; BALB/C (H-2$^d$) ; and C3H/HEN (H-2$^k$). It has been previously shown that only those mice of the H-2$^b$ haplotype produce antibodies against the repetitive epitope of the P. falciparum circumsporozoite protein. The antigen was injected with Freund's adjuvant and serum samples from immunized animals screened in an enzyme-linked immunoadsorbant assay (ELISA). Control animals were immunized with R32tet32, an antigen containing repeating tetrapeptides of the CS protein. Inoculation of all three mouse strains with $NSI_{81}RLf\Delta9$ resulted in the production of antibody reactive with the repeatless (flanking) region of the circumsporozoite protein. Details and results of the immunogenicity assay follow below.

Each of the three mouse strains was separated into two groups, each group comprising four to five animals. The first group of animals was immunized with $NSI_{81}RLf\Delta9$ and the second group immunized with R32tet32 (J. Young et al., Science 228 : 958 (1985)). R32tet32 contains two Xho II fragments, each fragment encoding a peptide having the sequence [(Asn-Ala-Asn-Pro)$_{15}$ (Asn-Val-Asp-Pro)]$_2$. tet$_{32}$ is a 32 amino acid peptide encoded by the tetracycline resistance gene, read out of frame.

$NSI_{81}RLf\Delta9$ antigen, purified according to Example 6, was mixed with complete Freund's adjuvant just prior to administration. Mice (6 to 8 weeks old) were each immunized subcutaneously with 50 μg. of antigen, administered in a 200 μl. dose to the right hind quarter. Mice were immunized twice, the first time with a single 200 μl. dose containing 50 μg. antigen in complete Freund's adjuvant. Booster injections were given four weeks later according to the same protocol as was used for the first injections except that the antigen was emulsified in incomplete Freund's adjuvant.

Animals were bled at 7 days following the second immunization. Whole blood from all animals within a group was pooled, clotted overnight at 4°C, and centrifuged to separate the serum which was then stored at -70°C. An ELISA was used to test the pooled sera for antibody produced against R32tet$_{32}$, $NSI_{81}RLf\Delta9$ or R16CSP. (R16CSP was prepared and purified as described above.)

The "sandwich" ELISA incorporated R32tet$_{32}$, R16CSP and $NSI_{81}RLf\Delta9$ as capture antigens adsorbed to the well walls of a microtitration plate.

The capture antigen was adsorbed to the well walls of the microtitration plate by adding to wells 50 μl. of a PBS solution containing 0.75 μg. of capture antigen and 0.2 μg. of boiled Casein. This solution was prepared by adding 8 μl. (3.76 μg.) of capture antigen to 2.5 ml. of a solution consisting of 4 μl. of a 0.5% boiled Casein solution (described below) to 5 ml. of Dulbecco's phosphate-buffered saline (PBS) (an aqueous solution comprising 0.8% NaCl ; 0.217% $Na_2HPO_4$-$7H_2O$ ; 0.02% $KH_2PO_4$ ; and 0.02% KCl, having a ph of 7.4).

After overnight incubation at room temperature, the well contents were aspirated and the remaining active binding sites on the plates blocked with a boiled 0.5% Casein solution (5 g/l. Casein (J.T. Baker Chemical Co.); 0.1 g/1. Thimersol (Sigma Chemical Co.) ; 0.02 g/l. phenol red (Sigma Chemical Co.) ; 900 ml. PBS, pH 7.4 ; and 100 ml. 0.1 N NaOH) in admixture (99 : 1) with 1% Tween 20 (polyoxyethylenesorbitan monolaurate, Sigma Chemical Co.).

Mouse sera samples were diluted 1 : 100 ; 1 : 1000 ; 1 : 10,000 ; 1 : 100,000 and 1 : 1,000,000 in a 0.5% boiled Casein solution containing 0.025% Tween 20. The test serum was then added to the well and after 2 hours incubation, the serum removed and the wells washed twice with a PBS solution containing 0.05% Tween 20. An anti-mouse IgG Ab conjugated to peroxidase, diluted 1 : 2000 with the same diluent used for sera, was then added to the well. After one hour incubation, the well contents were aspirated, washed 3 times with the PBS solution containing 0.05% Tween 20, and a clear peroxidase substrate solution (Kirkegaard & Perry, prepared according to manufacturer's instructions) added. Reaction of the peroxidase with the substrate resulted in the formation of a dark green product, the intensity of the color being proportional to the amount of antibody present in the serum sample. Results were read after 15 minutes at 405 to 414 nanometers using an ELISA plate reader and recorded in Optical Density (O.D.) units.

Innoculation of all three mouse strains C3H/HEN (H-$2^k$), BALB/C (H-$2^d$) and C3H/HEN (H-$2^b$) with the NSI$_{81}$RLfΔ9 antigen resulted in the formation of antibodies which reacted strongly to the repeatless region of the circumsporozoite protein. Reactivity to the NSI$_{81}$RLfΔ9 capture antigen was only slightly greater than that to R16CSP. (R16CSP capture antigen will detect antibodies only to the repeatless portion of the CS protein whereas the NS1$_{81}$RLfΔ9 capture antigen permits detection of both anti-NSI$_{81}$ and anti-RLfΔ9 antibodies). As expected, C3H/HEN mice did not raise an antibody response to R32tet$_{32}$ whereas C57Bl/6 mice did. While significantly weaker (1 log difference) than the antibody response observed in C57BL/6 mice, the antibody response of the BALB/C mice to R32tet$_{32}$ was not expected and is contrary to the negative response reported in the literature for mice of this haplotype to (NANP)$_{40}$(Del Giudice, et al., J. Immunol. 137 : 2952 (1986) reporting that out of fourteen strains of mice bearing nine different H-2 haplotypes, including BALB/C (H-$2^d$), immunized with (NANP)$_{40}$ without a carrier protein, only H-$2^b$ mice mounted an antibody response against (NANP)$_{40}$. H-$2^d$ mice (BALB/C) did not respond at all. BALB/C mice immunized with (NANP)$_{40}$ coupled to keyhole limpet hemocyanin as a carrier protein did raise anti-(NANP)$_{40}$ antibodies).

## EXAMPLE 9

### Inhibition of Sporozoite Invasion (ISI),

In this study, serum of rabbits immunized with NS1$_{81}$RLfΔ9 was tested for its ability to inhibit the entry of P. falciparum sporozoites into liver cells, the site of sporozoite development and maturation into the exo-erythrocytic stage.

Three mouse strains, BALB/C, C57BL/6 and A/J, immunized with NS1$_{81}$RLfΔ9, administered in either complete Freund's adjuvant or aluminum hydroxide, developed high antibody titers to the repeatless region of the CS protein. However, sera of these mice was unable to block invasion of sporozoites into cultured human hepatoma cells (HepG2-A16) when assayed according to the Inhibition of Sporozoite Invasion (ISI) assay described in Hollingdale, M.R. et al., J. Immunol, 132(7) : 909 (1984).

In contrast, New Zealand white rabbits, immunized with 100 μg. NS1$_{81}$RLfΔ9, administered in complete Freund's adjuvant at week 0, 3 and 7, elicited elevated antibody titers (albeit lower than that measured in mice) to the repeatless region of the CS protein. However, serum from rabbits immunized with NS1$_{81}$RLfΔ9 was demonstrated to significantly inhibit (98% in one animal, the average inhibition being about 60%) invasion of hepatoma cells by sporozoites when tested according to the Hollingdale ISI assay.

Sporozoites of a chloroquine resistant strain of P. falciparum (strain 7G8) and a chloroquine sensitive strain of P. falciparum (strain NF54) were each significantly inhibited from entering hepatoma cells by sera from rabbits immunized with NSI$_{81}$RLfΔ9. Inhibition of hepatoma invasion by sporozoites of P. falciparum strain 7G8 was higher (average 85%) than the ISI determined for strain NF54 (average 60%).

In ISI studies in which normal human hepatocytes were substituted for human hepatoma cells, P. falciparum strain NF54 sporozoites were inhibited (89% inhibition by serum from one rabbit, the average inhibition being about 45%) from entering hepatocytes by sera from rabbits immunized with NSI$_{81}$RLfΔ9.

These studies suggest the presence of sporozoite neutralizing epitopes on the NSI$_{81}$RLfΔ9 antigen.

## EXAMPLE 10

### Identification of Sporozoite Neutralizing Epitopes

Briefly, three mouse strains, BALB/C, C57BL/6 and A/J, immunized with NSI$_{81}$RLfΔ9, administered in either complete Freund's adjuvant or aluminum hydroxide, developed high antibody titers to the repeatless region of the CS protein. However, sera of these mice was unable to block invasion of sporozoites into cultured human hepatoma cells (HepG2-A16) when assayed according to the Inhibition of Sporozoite Invasion (ISI) assay described in Hollingdale, M.R. et al. J. Immunol, 132(7) : 909 (1984).

The mouse and rabbit sera was then further analyzed by the pepscan analysis of Geysen et al., Strategies for Epitope Analysis Using Peptide Synthesis, " J. Immunol. Methods 102 : 259-274 (1987). Briefly, a complete set of overlapping hexapeptides, homologous with the sequence of the complete P. falciparum CS protein sequence, were synthetically prepared, each hexapeptide being coupled to a polypropylene rod. Sera from mice and rabbits immunized as described in Example 9 with RLfΔ9, was diluted 1 : 500 and incubated with the rods at 4°C overnight. The rods were washed repeatedly and bound antibodies were detected by alkaline phosphatase conjugated to anti-species antibodies. After several washes, the rods were exposed to substrate for one hour. Absorbance was read at 414 nm and results were plotted to the appropriate pin with its associated sequence. Analysis of the hexapeptide responses permitted the identification of the five sporozoite neutralizing epitopes described below.

The following peptides of the invention were identified, using the methods described above, as sporozoite neutralizing epitopes of P. falciparum.

Peptide 1

Gly-Asp-Asn-Gly-Arg-Glu-Gly-Lys

Peptide 2

Glu-Lys-Leu-Arg-Lys-Pro-Lys

Peptide 3

Leu-Lys-Gln-Pro-Gly-Asp-Gly-Asn

Peptide 4

Pro-Asn-Asp-Pro-Asn-Arg-Asn-Val-Asp-Glu-Asn-Ala

Peptide 5

Asn-Lys-Pro-Lys-Asp-Glu-Leu-Asp

Any of Peptides 1-5 can be prepared via conventional synthesis techniques using the guidance provided in this application.

Any of Peptides 1 to 5 can be coupled to an immunoenhancing macromolecule, such as, but not limited to, tetanus toxoid, diptheria toxin and cholera B toxin. Preferably the coupling is mediated via an amino acid residue which aids in the coupling of peptides to carrier proteins, such as, but not limited to, cysteine, tyrosine and lysine. Also preferably the coupling is mediated via a coupling agent, i.e., an agent which bridges the peptide to the carrier protein. Useful coupling agents include, but are not limited to, glutaraldehyde. Most preferably, the coupling is mediated via a facilitator and a coupling agent.

For example, Peptide 4 was synthesized with a cysteine at its N-terminal and the resulting sequence was coupled, via the cysteine residue at its N-terminal, to tetanus toxoid using glutaraldehyde as the coupling agent (the resulting hybrid protein is hereinafter referred to a peptide 101). Peptide 4 was also synthesized with a cysteine at both its N- and C-terminals, and the resulting sequence was coupled, via the cysteine residue at its N-terminal and the cysteine residue at its C-terminal, to tetanus toxoid using glutaraldehyde as the coupling agent (the resulting hybrid protein is hereinafter referred to as peptide 106).

For example, Peptide 4 (containing a cysteine at both its N- and C-terminals) was suspended in PBS, pH 7.4, at a concentration of 1 mg/ml. Tetanus toxoid was added to acheive a final concentration of 1 mg/ml. Gluteraldehyde was added to a final concentration of 3% while the peptide and protein were stirred. The reaction was allowed to procede at room temperature for 1 hour. The resulting hybrid protein (peptide 106) was dialyzed against PBS to remove free peptide and gluteraldehyde.

The resulting coupled peptides (i.e., peptides 101 and 106) were used to immunize rabbits using Freund's complete and incomplete adjuvant, and sera were tested by ISI. The results are presented below.

| SERUM | % ISI |
|---|---|
| Antipeptide 101 | 71 |
|  | 50 |
| Antipeptide 106 | 99.2 |
|  | 89 |

ISI was the percent reduction in sporozoite invasion by test sera compared to the percent reduction in the presence of preimmunization or non-immune sera. As can be seen from the above results, both peptide 101 and 106 clearly elicited ISI activity. Peptide 106 was especially active.

In a similar manner, any or all of peptides 1 through 5 can be coupled together in any order and/or in any combination thereof via conventional techniques and then coupled to an immunoenhancing macromolecule (such as tetanus toxoid), preferably via a amino acid residue (such as a cysteine residue) at the resulting N-terminal and/or C-terminal, and a coupling agent (such as glutaraldehyde). The resulting hybrid polypeptide is useful in the vaccine of the invention.

It is also important to note that any protein encoded by the amino acid sequence of any of peptides 1 through 5 can be produced by reverse translating the peptide, via conventional techniques, back into a DNA sequence so that the protein may be expressed via conventional recombinant techniques as a monomer or polymer. It is also important to note that any desired hybrid peptides derived from peptides 1 through 5 can be created by reverse translating the peptide, via conventional techniques, back into a DNA sequence so that, once reversed translated back into a DNA sequence, such sequence can be genetically fused to desirable immunoenhancing coding sequences (e.g., tetanus toxoid) and/or expression enhancing sequences via conventional techniques and then the resulting hybrid amino acid coding sequence may be expressed via conventional recombinant DNA techniques.

Thus, in certain embodiments, the polypeptide of the invention comprises one or more of the above-identified immunogenic determinants. It is contemplated that functional equivalents of the above-listed peptides, i.e., derivative molecules possessing sporozoite neutralizing activity, may be prepared by conventional techniques well-known in the art. For example, such equivalents may have single or multiple amino acid deletions, substitutions and or additions as compared to the identified amino acid sequence while retaining sporozoite neutralizing activity.

## Claims

1. A polypeptide comprising one or more immunogenic determinants from the first flanking region of a Plasmodium surface protein, one or mare immunogenic determinants from the second flanking region of a Plasmodium surface protein, and fewer than all or no repeating immunogenic determinants from the repeat domain therebetween, wherein at least one of said immunogenic determinants from the first flanking region is encoded by the amino acid sequence
Gly-Asp-Asn-Gly-Arg-Glu-Gly-Lys ;
Glu-Lys-Leu-Arg-Lys-Pro-Lys ; or
Leu-Lys-Gln-Pro-Gly-Asp-Gly-Asn ;
or a functional equivalent thereof.

2. A polypeptide comprising one or more immunogenic determinants from the first flanking region of a Plasmodium surface protein, one or more immunogenic determinants from the second flanking region of a Plasmodium surface protein, and fewer than all or no repeating immunogenic determinants from the repeat domain therebetween, wherein at least one of said immunogenic determinants from the second flanking region is encoded by the amino acid sequence
Pro-Asn-Asp-Pro-Asn-Arg-Asn-Val-Asp-Glu-Asn-Ala ; or
Asn-Lys-Pro-Lys-Asp-Glu-Leu-Asp ;
or a functional equivalent thereof.

3. A polypeptide comprising one or more immunogenic determinants from the first flanking region of a <u>Plasmodium</u> surface protein, one or more immunogenic determinants from the second flanking region of a <u>Plasmodium</u> surface protein, and fewer than all or no repeating immunogenic determinants from the repeat domain therebetween, wherein at least one of said immunogenic determinants from the first flanking region is encoded by the amino acid sequence
Gly-Asp-Asn-Gly-Arg-Glu-Gly-Lys ;
Glu-Lys-Leu-Arg-Lys-Pro-Lys ; or
Leu-Lys-Gln-Pro-Gly-Asp-Gly-Asn ;
and wherein at least one of said immunogenic determinants from the second flanking region is encoded by the amino acid sequence
Pro-Asn-Asp-Pro-Asn-Arg-Asn-Val-Asp-Glu-Asn-Ala ; or
Asn-Lys-Pro-Lys-Asp-Glu-Leu-Asp ;
or a functional equivalent thereof.

4. A polypeptide comprising fewer than all or no repeating immunogenic determinants from the repeat domain of a <u>Plasmodium</u> surface protein and at least one peptide encoded by the amino acid sequence :
Gly-Asp-Asn-Gly-Arg-Glu-Gly-Lys ;
Glu-Lys-Leu-Arg-Lys-Pro-Lys ;
Leu-Lys-Gln-Pro-Gly-Asp-Gly-Asn ;
Pro-Asn-Asp-Pro-Asn-Arg-Asn-Val-Asp-Glu-Asn-Ala ; or
Asn-Lys-Pro-Lys-Asp-Glu-Leu-Asp ;
or a functional equivalent thereof.

5. The polypeptide of any of Claims 1 to 4 which is a hybrid polypeptide.

6. The polypeptide of Claim 5 which is the result of chemical fusion of the peptide to a carrier protein.

7. The polypeptide of Claim 6 wherein the carrier protein is tetanus toxoid, diptheria toxin or Cholera B toxin.

8. The polypeptide of Claim 7 wherein the chemical fusion is mediated via glutaraldehyde as the coupling agent.

9. The polypeptide of Claim 7 wherein the fusion to tetanus toxoid is mediated via a cysteine residue at the N-terminal of the peptide.

10. The polypeptide of Claim 7 wherein the fusion to tetanus toxoid is mediated via a cysteine residue at the N-terminal and a cysteine residue at the C-terminal of the peptide.

11. The polypeptide of Claim 10 which is the result of the coupling of the amino acid sequence Cys-Pro-Asn-Asp-Pro-Asn-Arg-Asn-Val-Asp-Glu-Asn-Ala-Cys to tetanus toxoid via glutaraldehyde as the coupling agent.

12. An expression vector encoding the polypeptide of any of Claims 1-11.

13. A vaccine for protecting humans against infection by <u>Plasmodium</u> sporozoites comprising an immunoprotective amount of the polypeptide of any of Claims 1-11 and a pharmaceutically acceptable carrier.

14. A method of immunizing a human in need of protection against infection by <u>Plasmodium</u> sporozoites comprising administering an effective, non-toxic amount of the vaccine of Claim 13 to such human.

**European. Patent Office**

## PARTIAL EUROPEAN SEARCH REPORT

which under Rule 45 of the European Patent Convention shall be considered. for the purposes of subsequent proceedings. as the European search report

Application number

| | DOCUMENTS CONSIDERED TO BE RELEVANT | | EP 90313257.9 |
|---|---|---|---|
| Category | Citation of document with indication, where appropriate, of relevant passages | Relevant to claim | CLASSIFICATION OF THE APPLICATION (Int. Cl.¾) 5 |
| A | DE - A1 - 3 723 583 (ENIRICERCHE) * Claims 1,2,16 * | 1-4,13 | C 07 K 7/00 C 12 N 15/63 A 61 K 37/02 |
| A | EP - A1 - 0 191 748 (SMITHKLINE) * Claims 1-3; page 4, lines 10-22; page 8, lines 20-22 * | 1,5,6, 12,13 | |

**TECHNICAL FIELDS SEARCHED (Int. Cl.¾) 5**

C 07 K
C 12 N
A 61 K

## INCOMPLETE SEARCH

The Search Division considers that the present European patent application does not comply with the provisions of the European Patent Convention :: such an extent that it is not possible to carry out a meaningful search into the state of the art on :~ basis of some of the claims.

Claims searched completely: *1-13*

Claims searched incompletely: —

Claims not searched: *14(Article 52(4))*

Reason for the limitation of the search:

| Place of search | Date of completion of the search | Examiner |
|---|---|---|
| VIENNA | 20-03-1991 | BÖHM |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family. corresponding document

EPO Form 1505.1 .03.82